**Europäisches Patentamt**

**European Patent Office**

**Office européen des brevets**

(19)

(11) Publication number: **0 209 037**
**B1**

(12) # EUROPEAN PATENT SPECIFICATION

(45) Date of publication of the patent specification:
28.02.90

(51) Int. Cl.⁴: **A61K 31/685**
// (A61K31/685, 31:35)

(21) Application number: 86109256.7

(22) Date of filing: 07.07.86

(54) Pharmaceutical compositions containing flavanolignanes and phospholipids as active principles.

(30) Priority: 17.07.85 IT 2160385

(43) Date of publication of application:
21.01.87 Bulletin 87/4

(45) Publication of the grant of the patent:
28.02.90 Bulletin 90/9

(84) Designated Contracting States:
AT BE CH DE FR GB IT LI LU NL SE

(56) References cited:
FR-A- 2 343 481
FR-M- 5 060

ROTE LISTE, 1974, Editio Cantor, Aulendorf, DE;
UNLISTED DRUGS, vol. 33, no. 3, March 1981, Chatham,
New Jersey, US;

(73) Proprietor: INVERNI DELLA BEFFA S.P.A., Via
Ripamonti, 99, I-20141 Milano(IT)

(72) Inventor: Bombardelli, Ezio, Via Ripamonti, 99,
I-20141 Milan(IT)
Inventor: Magistretti, Maria José, Via Ripamonti, 99,
I-20141 Milan(IT)

(74) Representative: Bianchetti, Giuseppe, Studio
Consulenza Brevettuale Via Rossini, 8, I-20122 Milan(IT)

## Description

The invention relates to novel pharmaceutical compositions containing a combination of two drugs, both used in the treatment of liver diseases, a) flavanolignans and b) phospholipids. More particularly, the invention relates to pharmaceutical compositions containing a combination of silymarin and phospholipids with a high content of polyunsaturated acyl chains. (It is well-known that silymarin contains three main constituents, i.e. the flavanolignans silybin, silydianin and silychristin; this invention relates to pharmaceutical compositions containing, as a) component, both silymarin as such and one or more of the three above mentioned flavanolignans, even though the invention will refer only to silymarin, for the sake of simplicity).

Silymarin is known to exert a stabilizing action on the liver-cell membrane against the poisoning effect of some injurious agents such as phalloidine, amanitine, carbon tetrachloride, galactosamine, and is widely employed in the treatment of liver diseases of various origin.

Phospholipids (particularly phosphatidylcholine, which is the main constituent of all the endogenous phospholipids) are essential for the normal structure and function of the liver cells, intervening in the regulation of the fluidity and permeability of the cell walls.

Each liver disease, even of viral origin, involves a membrane damage resulting from an impaired synthesis of the phospholipids or a peroxidation of the same, with a consequent lysis of the walls, and necrosis of the tissue.

Phospholipids, that are absorbed as such also in the gastroenteric tract (Porcellati), act directly on the liver-cell membrane, wherein they are incorporated to balance the phospholipids deficiency.

The present invention shows that the combination of silymarin (which inter alia stimulates the in vivo biosynthesis of phosphatidylcholine) with exogenous phospholipids, particularly with unsaturated exogenous phospholipids from soy or liver, is more active than the single components in the prevention and treatment of liver disease. Surprisingly, the activity of such a combination takes place at lower doses than those usually used for the components; the weight ratio between silymarin and phospholipids may vary between 1:0.5 and 1:5, preferably the a:b weight ratio is from 1:1 to 1:3.

Particularly preferred combinations according to the invention contain a) 1 part (by weight) of silymarin containing 80% of flavanolignans, with a weight ratio silybin:silydianin:silychristin = 3:1:1, and b) 2 parts (by weight) of phosphatidylcholine having a high content of polyunsaturated acyl groups. Among these combinations, particularly preferred is the one in which the b) component is a soy phosphatidylcholine wherein the acyl groups consist of at least 60% linoleic acid groups. Likewise preferred are the combinations in which liver phospholipids replace soy phosphatidylcholine, said liver phospholipids being particularly rich in linolenic acid. Non limiting examples of such a phosphatidylcholine are a soy-bean phosphatidylcholine (known as "Lipoid® S 100"), having a minimum content of 90% phosphatidylcholine, containing on average 63% linoleic acid, 16% palmitic acid, 11% oleic acid and 3.5% stearic acid out of the total fatty acids; or a phosphatidylcholine containing 80% linoleic acid, 5% linolenic acid and 15% oleic acid ("EPL Nattermann").

The following pharmacological tests show the activity of the new compositions and the advantages in comparison with the single components. Table 1 shows the liver protection obtained in the conventional test of liver poisoning with $CCl_4$, in the rat. Table 2 shows the protection obtained in the liver poisoning with praseodymium, in the rat, by the administration of silymarin, phosphatidylcholine and, respectively, combinations thereof.

TABLE 1

POISONING WITH CCl$_4$ (20% IN PARAFFIN OIL) (3ml/kg OS)

| Treatment | Dose mg/kg os x 3d | Weight of the liver (g) | GPT U/L | GOT U/L |
|---|---|---|---|---|
| controls | - | 5.52 ±000 0.21 | 25.67±000 2.42 | 90.75±000 7.90 |
| controls | - | 7.82 + 0.17 | 202.25+ 18.13 | 286.92+ 27.63 |
| Silymarin + phosphatidylcholine | 50+ 75 | 6.27 ±000 0.22 (-20) | 60.75+000 7.19 (-70) | 127.50+000 13.32 (-56) |
| Silymarin | 50 | 7.21 ±0 0.16 (-8) | 114.58+00 24.61 (-43) | 190.25+0 31.60 (-34) |
| Phosphatidylcholine | 75 | 7.29 ±0 0.17 (-7) | 112.33+00 24.09 (-44) | 200.17+0 18.71 (-30) |

1) No. 12 animals per group

o     <0.05

oo    <0.01 Vs. controls CCl$_4$

ooo   <0.001

## TABLE 2

### POISONING WITH PRASEODYMIUM (10 mg/kg i.v.)

| Treatment | Dose mg/kg os [1] | GOT U/L | GPT U/L | Weight of liver (g) | Triglycerides mL/100 g |
|---|---|---|---|---|---|
| controls | – | 39.90 ± 1.93 [ooo] | 15.80 ± 0.73 [ooo] | 7.77 ± 0.14 [ooo] | 586.81 ± 67.18 [ooo] |
| Praseodymium controls | – | 235.89 ± 16.36 | 159.89 ± 11.67 | 9.44 ± 0.32 | 1121.18 ± 90.16 |
| Silymarin + phosphatidylcholine | 50 + 75 | 120.30 ± 10.11 [ooo] (–49%) | 98.35 ± 9.35 [ooo] (–38%) | 7.50 ± 0.15 [ooo] (–21%) | 720.30 ± 50.21 (–36%) |
| Silymarin | 50 | 167.30 ± 16.02 [o] (–29%) | 120.13 ± 10.30 [o] (–25%) | 8.52 ± 0.12 (–10%) | 980.15 ± 80.32 (–13%) |
| Phosphatidylcholine | 75 | 170.21 ± 15.13 (–28%) | 130.50 ± 9.55 (–18%) | 8.90 ± 0.13 (–6%) | 1080.81 ± 90.20 (–4%) |

1) Twice a day for two days + once the third day
2) No. 10 animals per group

o < 0.05; oo < 0.01; ooo < 0.001 Vs. praseodymium controls

The compositions according to the invention show no toxic effects. They can be used in form of pills, capsules, packets, or as aqueous suspensions, suitably flavoured.

The following examples illustrate some pharmaceutical compositions of the invention.

## EXAMPLE 1

### Chewable tablets containing silymarin and phosphatidylcholine

Tablets containing 200 mg of silymarin and 300 mg of phosphatidylcholine, with a high content of polyunsaturated fatty acids (Lipoid S 100) are prepared.

EP 0 209 037 B1

They have the following formula:
each 3 g tablet contains:
Silymarin 200 mg
Lipoid® S 100 (phosphatidylcholine) 300 mg
Ammonium glycyrrhizinate 15 mg
Sodium saccharine 10 mg
Talc 30 mg
Magnesium stearate 20 mg
Silica powder 30 mg
Orange juice (dried) 350 mg
Flavouring 35 mg
Fructose 1495 mg
Dextrose 515 mg

## EXAMPLE 2

Chewable tablets containing silymarin and phosphatidylcholine

Chewable tablets containing 200 mg of silymarin and 150 mg of phosphatidylcholine, with a high content of polyunsaturated fatty acids (Lipoid® S 100) are prepared.
They have the following formula:
each 1.5 g tablet contains:
Silymarin 200 mg
Lipoid® S 100 (phosphatidylcholine) 150 mg
Ammonium glycyrrhizinate 15 mg
Sodium saccharine 10 mg
Silica powder 25 mg
Talc 15 mg
Magnesium stearate 10 mg
Orange juice 250 mg
Flavouring 30 mg
Dextrose 255 mg
Fructose 550 mg

## EXAMPLE 3

Granulate for suspension in water, containing silymarin and phosphatidylcholine

They have the following formula:
each 4 g packet contains:
Silymarin 400 mg
Lipoid® S 100 (phosphatidylcholine) 600 mg
Lactose 2000 mg
Mannitol 757.5 mg
Ammonium glycyrrhizinate 10 mg
Sodium saccharine 2.5 mg
Orange juice 200 mg
Flavouring 30 mg.

**Claims for the contracting States: BE, CH, DE, FR, GB, IT, LI, LU, NL, SE**

1. Pharmaceutical compositions with anti-hepatotoxic activity, containing as the active principle a combination of:
a) one or more flavanolignans, and
b) one or more phospholipids,
in which the a:b weight ratio is from 1:0.5 to 1:5, either alone or in association with other active principles.
2. Pharmaceutical compositions according to claim 1, containing, as a)-component, flavanolignans selected from silybin, silydianin, silychristin and mixtures thereof.
3. Pharmaceutical compositions according to claims 1 and 2, containing silymarin as a)-component.
4. Pharmaceutical compositions according to claims 1–3, containing, as b)-component, phospholipids with a high content of polyunsaturated acyl groups.
5. Pharmaceutical compositions according to claim 4, containing, as b)-component, phosphatidylcholine from soy beans or from liver.

5

6. Pharmaceutical compositions according to claims 1–5, in which the a:b weight ratio is from 1:1 to 1:3.

7. Pharmaceutical compositions according to the above claims, characterized in that they contain, as the active principle, a combination of a) 1 part in weight of silymarin and b) 2 parts in weight of phosphatidylcholine from soy beans.

8. Pharmaceutical compositions according to claim 7, in form of chewable tablets.

9. Pharmaceutical compositions according to claim 7, in form of hydrodispersible granulates.

**Claims for the contracting State: AT**

1. Process for the preparation of pharmaceutical compositions with anti-hepatotoxic activity, containing as the active principle a combination of:

a) one or more flavanolignans, and

b) one or more phospholipids, in which the a:b weight ratio is from 1:0.5 to 1:5, either alone or in association with other active principles.

2. Process according to claim 1, wherein the flavanolignans are selected from silybin, silydianin, silychristin and mixtures thereof.

3. Process according to claim 1, wherein silymarin is used as flavanolignans.

4. Process according to claims 1–3, wherein the phospholipids have a high content of polyunsaturated acyl groups.

5. Process according to claim 4, wherein the phospholipid is phosphatidylcholine from soy beans or liver.

6. Process according to claims 1–5, in which the a:b weight ratio is from 1:1 to 1:3.

7. Process according to claims 1–6, wherein silymarin and phosphatidylcholine from soy beans are used in a 1:2 weight ratio.

**Patentansprüche für die Vertragsstaaten: BE, CH, DE, FR, GB, IT, LI, LU, NL, SE**

1. Pharmazeutische Zusammensetzungen mit anti-leberzellenschädigender Wirkung, welche als aktiven Wirkstoff eine Kombination von

a) einem oder mehreren Flavanolignanen und

b) einem oder mehreren Phospholipiden, worin das Gewichtsverhältnis a:b von 1:0,5 bis 1:5 beträgt, allein oder zusammen mit anderen aktiven Wirkstoffen enthalten.

2. Pharmazeutische Zusammensetzungen nach Anspruch 1, welche als Komponente a) Flavanolignane enthalten, die ausgewählt sind unter Silybin, Silydianin, Silychristin und Gemischen davon.

3. Pharmazeutische Zusammensetzungen nach Anspruch 1 und 2, welche als Komponente a) Silymarin enthalten.

4. Pharmazeutische Zusammensetzungen nach den Ansprüchen 1 bis 3, welche als Komponente b) Phospholipide mit einem hohen Gehalt an polyungesättigten Acylgruppen aufweisen.

5. Pharmazeutische Zusammensetzungen nach Anspruch 4, welche als Komponente b) Phosphatidylcholine von Sojabohnen oder aus der Leber enthalten.

6. Pharmazeutische Zusammensetzungen nach den Ansprüchen 1 bis 5, worin das Gewichtsverhältnis a:b im Bereich von 1:1 bis 1:3 liegt.

7. Pharmazeutische Zusammensetzungen nach den voranstehenden Ansprüchen, dadurch gekennzeichnet, daß sie als aktiven Wirkstoff eine Kombination von

a) einem Gewichtsteil Silymarin und

b) 2 Gewichtsteilen Phosphatidylcholin aus Sojabohnen enthalten.

8. Pharmazeutische Zusammensetzungen nach Anspruch 7 in Form von kaubaren Tabletten.

9. Pharmazeutische Zusammensetzungen nach Anspruch 7 in Form von hydrodispersiblen Granulaten.

**Patentansprüche für den Vertragsstaat: AT**

1. Verfahren zur Herstellung von pharmazeutischen Zusammensetzungen mit anti-leberzellenschädigender Wirkung, welche als aktiven Wirkstoff eine Kombination von

a) einem oder mehreren Flavanolignanen und

b) einem oder mehreren Phospholipiden, worin das Gewichtsverhältnis a:b von 1:0,5 bis 1:5 beträgt, allein oder zusammen mit anderen aktiven Wirkstoffen enthalten.

2. Verfahren nach Anspruch 1, worin die Flavanolignane ausgewählt sind unter Silybin, Silydianin, Silychristin und Gemischen davon.

3. Verfahren nach Anspruch 1, worin als Flavanolignane Silymarin benützt wird.

4. Verfahren nach den Ansprüchen 1 bis 3, worin die Phospholipide einem hohen Gehalt an polyungesättigten Acylgruppen aufweisen.

5. Verfahren nach Anspruch 4, worin das Phospholipid Phosphatidylcholine von Sojabohnen oder aus der Leber ist.

6. Verfahren nach den Ansprüchen 1 bis 5, worin das Gewichtsverhältnis a:b im Bereich von 1:1 bis 1:3 liegt.

7. Verfahren nach den Ansprüchen 1 bis 6, worin Silymarin und Phosphatidylcholin von Sojabohnen in Gewichtsverhältnis von 1:2 benützt werden.

## Revendications pour les états contractants: BE, CH, DE, FR, GB, IT, LI, LU, NL, SE

1. Compositions pharmaceutiques douées d'activité anti-hépatotoxique, contenant comme principe actif une combinaison de:
   a) un ou plusieurs flavanolignanes, et
   b) un ou plusieurs phospholipides,
   dans laquelle le rapport en poids a:b est de 1:0,5 à 1:5, soit seule, soit en combinaison avec d'autres principes actifs.

2. Compositions pharmaceutiques selon la revendication 1, contenant, comme composant a), des flavolignanes choisis parmi la silybine, la silydianine, la silychristine et leurs mélanges.

3. Compositions pharmaceutiques selon les revendications 1 et 2, contenant de la silymarine comme composant a).

4. Compositions pharmaceutiques selon les revendications 1 à 3, contenant, comme composant b), des phospholipides à forte teneur en groupes acyles polyinsaturés.

5. Compositions pharmaceutiques selon la revendication 4, contenant, comme composant b), de la phosphatidylcholine extraite de soja ou de foie.

6. Compositions pharmaceutiques selon les revendications 1 à 5, dans lesquelles le rapport en poids a:b est de 1:1 à 1:3.

7. Compositions pharmaceutiques selon les revendications précédentes, caractérisées en ce qu'elles contiennent, comme principe actif, une combinaison de a) une partie en poids de silymarine et b) deux parties en poids de phosphatidylcholine extraite de soja.

8. Compositions pharmaceutiques selon la revendication 7, sous forme de tablettes à mâcher.

9. Compositions pharmaceutiques selon la revendication 7, sous forme de granules dispersables dans l'eau.

## Revendications pour l'état contractant: AT

1. Procédé pour la préparation de compositions pharmaceutiques douées d'activité anti-hépatotoxique, contenant comme principe actif une combinaison de:
   a) un ou plusieurs flavanolignanes, et
   b) un ou plusieurs phospholipides,
   dans laquelle le rapport en poids a:b est de 1:0,5 à 1:5, soit seule, soit en combinaison avec d'autres principes actifs.

2. Procédé selon la revendication 1, dans lequel les flavolignanes sont choisis parmi la silybine, la silydiane, la silychristine et leurs mélanges.

3. Procédé selon la revendication 1, dans lequel la silymarine est utilisée comme flavanolignane.

4. Procédé selon les revendications 1 à 3, dans lequel les phospholipides ont une forte teneur en groupes acyles polyinsaturés.

5. Procédé selon la revendication 4, dans lequel le phospholipide est de la phosphatidylcholine extraite de soja ou de foie.

6. Procédé selon les revendications 1 à 5, dans lequel le rapport en poids a:b est de 1:1 à 1:3.

7. Procédé selon les revendications 1 à 6, dans lequel la silymarine et la phosphatidylcholine extraite de soja sont utilisées dans le rapport en poids de 1:2.